# EUROPEAN PATENT APPLICATION

(11) **EP 2 716 306 A1**
(43) Date of publication of application: **09.04.2014**
(21) Application number: 12187437.4
(22) Date of filing: 05.10.2012
(51) Int. Cl.: A61L 27/28, A61L 27/30, C04B 35/628

(54) **Metal oxide functionalized by strontium**

(71) Applicant: Aarhus Universitet, 8000 Aarhus C (DK); Elos Medtech Pinol A/S, 3330 Gørløse (DK)
(72) Inventor: Foss, Morten, 8660 Skanderborg (DK); Andersen, Ole Zoffmann, 8000 Aarhus C (DK); Thomsen, Christian Schärfe, 2200 Copenhagen (DK)
(74) Representative: Plougmann & Vingtoft A/S

(57) **Abstract**

The invention relates to a method for incorporating alkali metals or alkaline earth metals in a metal oxide and a metal oxide product. Through contacting an aqueous solution comprising alkali metals or alkaline earth metals with a metal oxide body, drying said metal oxide body and heating the dried metal oxide body at temperature higher than 1000 °C a penetration of alkali metals or alkaline earth metals in the metal oxide body is achieved. Desired release profile of alkali metals or alkaline earth metals can therefore be obtained avoiding the drawback of the prior art.

## Description

### FIELD OF THE INVENTION

The present invention relates to a method for incorporating alkali metals or alkaline earth metals into metal oxides. The invention further relates to a metal oxide functionalized by alkali metals or alkaline earth metals.

### BACKGROUND OF THE INVENTION

The field of orthopaedic implants is quite large both in volume and turn-over. Only considering dental implants, more than 6 million are annually implanted around the world. Even though the success rate is high, there are still failure rates between 5-9 % causing discomfort and pain and also prolonged healing rates. Hence there is a need for new and improved biomaterials.

Ceramic materials, such as ZrO₂, are promising candidates due to the cosmetic advantages as compared to Titanium, i.e. they appear bright white instead of grey.

However ceramic materials have a lower degree of biocompatibility in terms of osseointegration.

Furthermore ceramic materials have the disadvantage that following sintering they reach a high level of hardness hindering further processing, e.g. shaping of the implant after sintering is very difficult.

Hence, a method of improving the degree of biocompatibility with respect to osseointegration and faster bone tissue ingrowth of ceramic materials, such as ZrO₂, would be advantageous.

Hence, an improved functionalized ceramic material for use as body implants would be advantageous.

### OBJECT OF THE INVENTION

It is an object of the present invention to provide new and improved biomaterials for body implants.

In particular it may be seen as an object of the invention to increase the degree of biocompatibility with respect to osseointegration and rate of bone ingrowth of ceramic materials such as ZrO₂.

In particular, it may be seen as an object of the present invention to provide a method for incorporating alkali metals or alkaline earth metals into metal oxides and a metal oxide functionalized by strontium that solves the above mentioned problems of the prior art.

It is a further object of the present invention to provide an alternative to the prior art.

### SUMMARY OF THE INVENTION

The above described object and several other objects are intended to be obtained in a first aspect of the invention by providing a method for incorporating alkali metals or alkaline earth metals in a metal oxide, the method comprising:
a) contacting an aqueous solution comprising alkali metals or alkaline earth metals with a metal oxide body; b) drying the metal oxide body; c) heating the dried metal oxide body at a temperature higher than 1000 °C.

Alkali metals or alkaline earth metals may refer to alkali metal elements or alkaline earth metal elements according to the periodic table. In some embodiments alkali metals or alkaline earth metals may refer to alkali metal ions or alkaline earth metal ions.

The aqueous solution comprising alkali metals or alkaline earth metals may contain the metals in the form of free ions or bound or comprised within molecular carriers, such as organic molecules surrounding the metals. Thus, the aqueous solution comprising alkali metals or alkaline earth metals may contain chemical complexes containing the metals either in the form of ions or in the form of elements.

The invention is directed to a method for incorporating alkali metals or alkaline earth metals in a metal oxide used as a body implant so as to optimize the tissue response to the implant, e.g. by stimulating healing, such as tissue healing, in the vicinity of the implant. The alkali metals or alkaline earth metals used may have bone growth initiating or stimulating properties. Thus the presence of alkali metals or alkaline earth metals stimulates bone regeneration in the vicinity of the implant. When the implant is a dental implant the alkali metals or alkaline earth metals may also have tissue growth initiating or stimulating properties.

The specific properties of the implant produced by the method of the invention are influenced by the ability of a sustained release of alkali metals or alkaline earth metals in the vicinity of the implant.

In some embodiments the alkaline earth metal is strontium.

Indeed Strontium has healing properties, e.g. increase the activity of bone forming cells and improve implant fixation due to stimulated bone regeneration. However Strontium may be toxic if released in high concentration. In search of a solution to this problem the inventors devised the current invention.

In some embodiments the aqueous solution comprising alkali metals or alkaline earth metals, contains alkali metals or alkaline earth metal salts.

For example as the alkaline earth metal may be strontium these salts may be Sr salts, such as SrCl₂, Sr(OH)₂.

In some other embodiments these salts may be lithium or magnesium salts, such as LiCl or MgCl₂.

In some embodiments the metal oxide is a ceramic material, such as ZrO₂

The method is characterized by three steps.

The first step, i.e. contacting an aqueous solution comprising alkali metals or alkaline earth metals with a metal oxide body may be achieved, in some embodiments, by providing the aqueous solution comprising alkali metals or alkaline earth metals to at least one surface of the metal oxide body so that the aqueous solution comprising alkali metals or alkaline earth metals is in liquid contact with the at least one surface of the metal oxide body.

Contacting an aqueous solution comprising alkali metals or alkaline earth metals with a metal oxide body may be achieved, in some other embodiments, by submerging, such as by soaking, the metal oxide body in the aqueous solution.

In some embodiments the aqueous solution comprising alkali metals or alkaline earth metals with the metal oxide body comprises keeping the aqueous solution comprising alkali metals or alkaline earth metals in contact with the metal oxide body for a period of time between 1 sec and 48 hours. Preferably the period of time is between 1 sec and 24 hours. Depending on the pressure, the contact time needed for infiltration may vary, e.g. under low pressure/vacuum 1-5 seconds may be sufficient.

In some embodiments the concentration of alkali metals or alkaline earth metals in the aqueous solution comprising alkali metals or alkaline earth metals is between 0.001 M and 2M at room temperature.

Room temperature is defined in general term as describing the temperature of 20 °C at sea level pressure, i.e. standard atmospheric pressure 1,013 bars.

In some other embodiments the concentration of alkali metals or alkaline earth metals in the aqueous solution comprising alkali metals or alkaline earth metals is between 0.01M and 1M at room temperature.

In some embodiments the alkaline earth metals is in a salt form, such as SrCl₂, thus the concentration in said aqueous solution containing Sr ions is between 0.001 M and 2M at room temperature.

Solubility of salts lies within the person skilled in the art, i.e. higher temperature of the solution allows for higher salts concentration. Solubility may be increased by increasing temperature, e.g. solubility may increase around a factor of 2 when going from room temperature to 100 °C.

Thus the concentration of alkali metals or alkaline earth metal salts in said aqueous solution comprising alkali metals or alkaline earth metals may be between 0.002M and 4M at 100 °C, at standard atmospheric pressure.

In general, higher concentration of salts may be beneficial to the process of infiltration of alkali metals or alkaline earth metals, thus higher concentration of salts may be advantageous.

Salt with higher solubility in the relative solvents, such as aqueous solutions may therefore be used to improve the infiltration of the alkali metals or alkaline earth metals in the metal oxide.

Increasing the temperature of the solution may increase the concentration of the solution and therefore may also reduce the time needed for infiltration in the metal oxide body of the alkali metals or alkaline earth metals and in turn may reduce the contact time, between the aqueous solution and the surface of the metal oxide body, which is needed to obtain the desired infiltration.

In some further embodiments the step of contacting the aqueous solution comprising alkali metals or alkaline earth metals with the metal oxide body comprises keeping said metal oxide body in liquid contact with the aqueous solution comprising alkali metals or alkaline earth metals under reduced pressure, such as low vacuum. For example reduced pressure may be 0.1 bars or lower. In some other example reduced pressure may be rough vacuum or coarse vacuum such as between 760 and 25 torr or 100 and 3 KPa.

By allowing infiltration of the aqueous solution in the metal oxide body before the sintering step, optimal diffusion of the alkali metals or alkaline earth metals within the metal oxide body can be achieved also due to the high porosity of the metal oxide body before the sintering.

In some embodiments the alkali metals or alkaline earth metals may be forced into the metal oxide body by the use of an electrical field to force positive metal ions into the structure without them being reduced on the surface of the material. In this case it is likely that the movement of these ions would be restricted (restriction of the electrophoretic mobility) by the porous nature of the material. This would cause an up-concentration of the positive metal ion in the material. The second step, i.e. drying the metal oxide body, may be achieved, in some embodiments, by drying the at least one surface of the metal oxide body that was in liquid contact with the aqueous solution comprising alkali metals or alkaline earth metals.

The drying may be achieved by blowing a stream of nitrogen over the metal oxide body.

The drying may also be achieved by blowing a stream of nitrogen over the at least one surface of the metal oxide body that was in liquid contact with the aqueous solution comprising alkali metals or alkaline earth metals.

In some embodiments the drying further comprises placing the metal oxide body that was in liquid contact with the aqueous solution comprising alkali metals or alkaline earth metals under reduced pressure. The liquid contact may occur for a period between 1 sec and 100 minutes; preferable between 1 sec and 15 minutes. The reduced pressure may be a low vacuum condition, for example rough vacuum or coarse vacuum, such as between 760 and 25 torr or 100 and 3 KPa.

The step of drying may further comprise a low temperature heating step, e.g. in a temperature range between 25 and 350 °C.

This low temperature heating step has the function of removing any trace of solvent from the metal oxide body. It may be so that also molecular carriers are burned during the low temperature heating step, therefore be freeing the alkali metals or alkaline earth metals already during the drying step, i.e. during the low temperature heating step.

The third step, i.e. the heating step, has the function of sintering the metal oxide body.

In some embodiments during the heating step the molecular carriers for metals, such as organic molecules may be burned-off due to the high temperature involved and thus be freeing the metals within the metal oxide structure.

The heating step may occur in the temperature range between 1000 and 1500 °C. Thus, said heating step may be referred to as high temperature heating step as to distinguish it from the low temperature heating step potentially occurring during the drying step.

In some embodiments the method further comprises: a1) washing, e.g. rinsing, the metal oxide body that was in liquid contact with the aqueous solution comprising alkali metals or alkaline earth metals with water or with a second aqueous solution, thereby removing any residual alkali metals or alkaline earth metals based compound that might have been deposited onto the surface of the metal oxide body.

This step occurs before the drying step and after the contacting step.

In some other embodiments this step occurs after the high temperature heating step to remove eventual segregations of Sr to the surface.

In some other embodiments the method according to the first aspect of the invention further comprises: d) rinsing said metal oxide body, following the heating step.

The washing, e.g. rinsing, ensure that substantially no alkali metals or alkaline earth metals are present on the external surface of the metal oxide body.

The inventors devised the invention in observing potential toxicity of alkali metals or alkaline earth metals deposited on the external surface of the metal oxide body. Thus, removal of surface deposition of alkali metals or alkaline earth metals has the advantage of avoiding potential local toxic concentrations of alkali metals or alkaline earth metals.

Indeed the method of the invention allows for a desired release rate of alkali metals or alkaline earth metals. As the alkali metals or alkaline earth metals are not deposited on the surface of the metal body their potential toxicity, due to fast release, or release into a too high concentration is avoided. The deep infiltration of the alkali metals or alkaline earth metals thus allows for a controlled released of the metals, e.g. in the form of metal ions, thus insuring optimal concentration release and tuneable release rate.

The method according to any of the preceding claims wherein said metal oxide body is a body implant.

Body implant is defined as a medical device manufactured to replace a missing biological structure, support a damaged biological structure, or enhance an existing biological structure. A dental implant may be considered a body implant. Orthopaedic implants may also be considered body implants.

In a second aspect the invention relates to a ceramic body produced by the method according to the first aspect of the invention.

In a third aspect a ceramic body is provided comprising a metal oxide body comprising alkali metals or alkaline earth metals in the metal oxide body, wherein the concentration of the alkali metals or alkaline earth metals in the bulk is higher than the one at the external surface of the ceramic body.

In some embodiments according to the second and third aspect of the invention the concentration in the bulk is between 2 and 10 times higher than the one at the external surface of the ceramic body.

The concentration in the bulk, i.e. bulk concentration is defined herein as within 10 µm from the external surface of the metal oxide body.

The external surface is herein defined within the outer 100 nm of the ceramic body.

The concentration at the external surface may be between 0.01 % and 0.1 and the concentration in the bulk, i.e. with 10 µm, is between 0.02 and 1%.

As percentage (%) is herein referred to as atomic percentage.

In some other embodiments the concentration at the external surface is 0.01 % and the concentration in the bulk, i.e. with 10 µm, is 0.07%.

As alkaline earth metals may be Strontium, the concentration of Sr at the surface of the ceramic body may be lower than the concentration in the bulk of the ceramic body.

In some embodiments according to the second and third aspect of the invention, the ceramic body comprises a metal oxide body comprising alkali metals or alkaline earth metals in the metal oxide body having a structure characterized by a concentration gradient of the alkali metals or alkaline earth metals throughout the metal oxide body.

In a fourth aspect of the invention a ceramic body is provided, the ceramic body comprising alkali metals or alkaline earth metals in the metal oxide body having a structure characterized by a concentration gradient of the alkali metals or alkaline earth metals throughout said metal oxide body.

As alkaline earth metals may be Strontium, the ceramic body may comprise: a metal oxide body comprising Sr having a structure characterized by a concentration gradient of the Sr throughout the metal oxide body.

In a fifth aspect a ceramic body is provided comprising: a metal oxide body comprising Sr having a structure characterized by a concentration gradient of the Sr throughout the metal oxide body.

The ceramic body may be referred to as body implant such as a dental implant.

In a sixth aspect a method for incorporating alkali metals or alkaline earth metals in a metal oxide is provided, the method comprising: providing a metal oxide powder comprising binding media; adding alkali metals or alkaline earth metals containing compounds to the metal oxide powder, thereby producing a mixture; processing the mixture, such as applying pressure and/or heat to the mixture, thereby producing a body comprising the mixture; applying heat to the body. Alkali metals or alkaline earth metals containing compounds is defined as any compounds comprising alkali metals or alkaline earth metals. For example these compounds may contain the metals in the form of free ions or bound or comprised within molecular carriers, such as organic molecules surrounding the metals.

As the step of applying heating is at high temperature, any organic compounds comprising alkali metals or alkaline earth metals may be burned-off due to the high temperature involved and thus be freeing the metal within the metal oxide structure.

Due to the temperature involved in this heating step it may also be referred to as a sintering step.

As alkaline earth metals may be Strontium in a further aspect of the invention a method for incorporating Strontium in a metal oxide is provided, the method comprising: providing a metal oxide powder comprising binding media; adding a Sr containing compound to the metal oxide powder, thereby producing a mixture; processing the mixture, such as applying pressure and/or heat to the mixture, thereby producing a body comprising the mixture; applying heat to the body. The metal oxide body may be produced by means of additive manufacturing. Additive manufacturing or 3D printing is a process of making three dimensional solid objects from a digital model. 3D printing is achieved using additive processes, where an object is created by laying down successive layers of material. 3D printing is considered distinct from traditional machining techniques (subtractive processes) which mostly rely on the removal of material by drilling, cutting and other mechanical steps.

In some further embodiments the metal oxide body production lies within the person skilled in the art.

The first, second and other aspects and embodiments of the present invention may each be combined with any of the other aspects and embodiments. These and other aspects and embodiments of the invention will be apparent from and elucidated with reference to the embodiments described hereinafter.

### BRIEF DESCRIPTION OF THE FIGURES

The method and ceramic body according to the invention will now be described in more detail with regard to the accompanying figures. The figures show one way of implementing the present invention and is not to be construed as being limiting to other possible embodiments falling within the scope of the attached claim set.
Figure 1 shows the strontium release profiles from two different zirconium oxide implant bodies alongside the release profiles from three different titanium implants that have been coated with a titanium oxide layer comprising Sr.
Figure 2a is a top view of the sample section used for the Rutherford backscattering (RBS) analysis. The dark square, approx. 1.5x1.5 mm² indicates the position from which the spectrum was acquired.
Figure 2b is a side view of the sample used for the RBS analysis. The area of spectrum acquisition is located on the surface exposed during cutting of the sample.
Figure 3 is a graphic plot of data from the RBS measurement and simulation.
Figure 4 is another graphic plot of data from the RBS measurement and simulation.
Figure 5 is a flow-chart of a method according to the invention.

### DETAILED DESCRIPTION OF AN EMBODIMENT

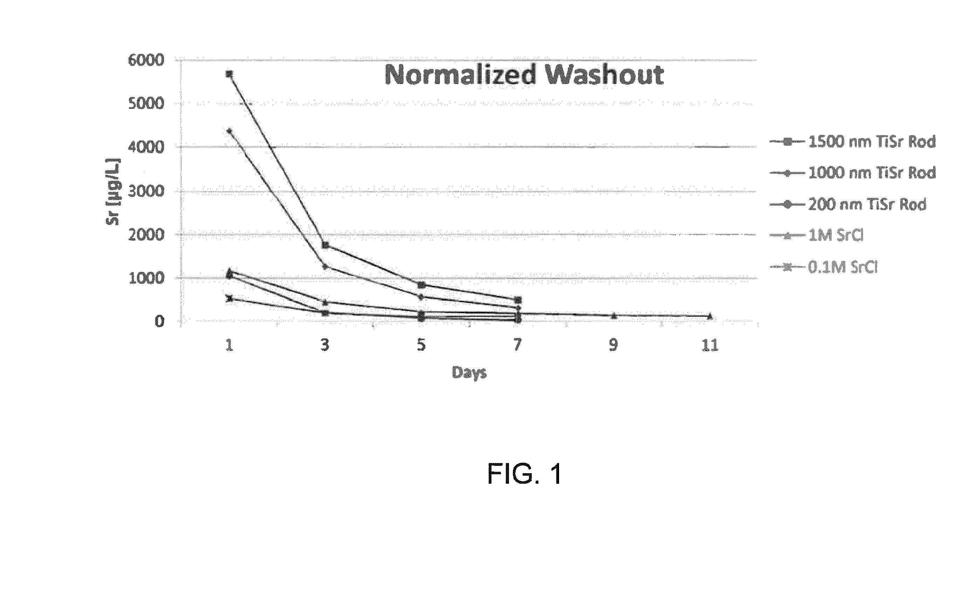

Figure 1 shows the normalized (same surface area) strontium release profiles from two different zirconium oxide implant bodies (0.1M SrCl and 1M SrCl) alongside the release profiles from three different titanium implant (1500 nm TiSr rod, 1000 nm TiSr rod and 200 nm TiSr rod), that have been coated with an oxide layer comprising Sr. The profiles has been obtained by Inductively coupled plasma optical emission spectroscopy (ICP-OES)

The ICP-OES studies of Sr release profiles on the samples have been performed in correlations with in vivo data from a rodent study where the three different titanium implants that have been coated with an oxide layer comprising Sr have been implanted.

0.1M SrCl and 1M SrCl refer to ZrO₂ prepared following the method of the invention from aqueous solution 0.1M SrCl₂ and 1M SrCl₂ respectively.

1500 nm TiSr rod, 1000 nm TiSr rod and 200 nm TiSr rod refer to titanium rods coated with a metal oxide layer comprising Sr having thicknesses of 1500, 1000 and 200 nm respectively.

The first two samples, i.e. 0.1M SrCl and 1M SrCl have been produced following the procedure below.

Unsintered ZrO₂ rods were washed in milliQ water and submerged in an aqueous solution of strontium chloride (SrCl₂) at concentrations between 0.1 M and 1 M. The submerged samples were kept in open containers and placed under vacuum for a period of 24 hours. At this point the samples were retrieved and given a quick wash to remove any residual strontium that might have precipitated onto the surface of the rod. The rods were then dried under a stream of nitrogen and, in order to remove any water trapped in the material, placed under low vacuum conditions for a period of 15 minutes.

At this point the samples were sintered at a temperature above 1000°C.

The sintered samples were subjected to washout test by ICP-OES in order to assess the Sr release profile.

In a previous rodent study related to the three different TiSr implants in figure 1, it was found that i) inflammation occurred in vivo in relation with some of the inserted implants, ii) there was a positive effect on bone ingrowth for the thickest (1500 nm) coating. The inflammation is most likely related to the burst release of Sr observed especially with thick coatings.

In search for a more constant release of Sr in relation with the initial osseointegration and long term stability of the implant the inventors devised the present invention. Hence, a lowering of the Sr release on day 1 while maintaining a relatively high Sr release on later time points was achieved.

The release profiles of the two ZrO₂ samples are more desirable as compared to those of the Ti/Sr implants, both in terms of the initial washout and stability of the long term release rates. It appears possible to further tailor the release profiles of the ZrO₂ implants by changing e.g. the temperature during the SrCl₂ incubation step. This would allow for a higher concentration of SrCl₂ in the solution leading to an increased uptake of ions in the ZrO₂ structure and ultimately an increase in the amount of Sr released from the body implant.

Other salts or combinations of Sr carrier are possible e.g. Strontium hydroxide, Lithium Chloride or/and Magnesium Chloride.

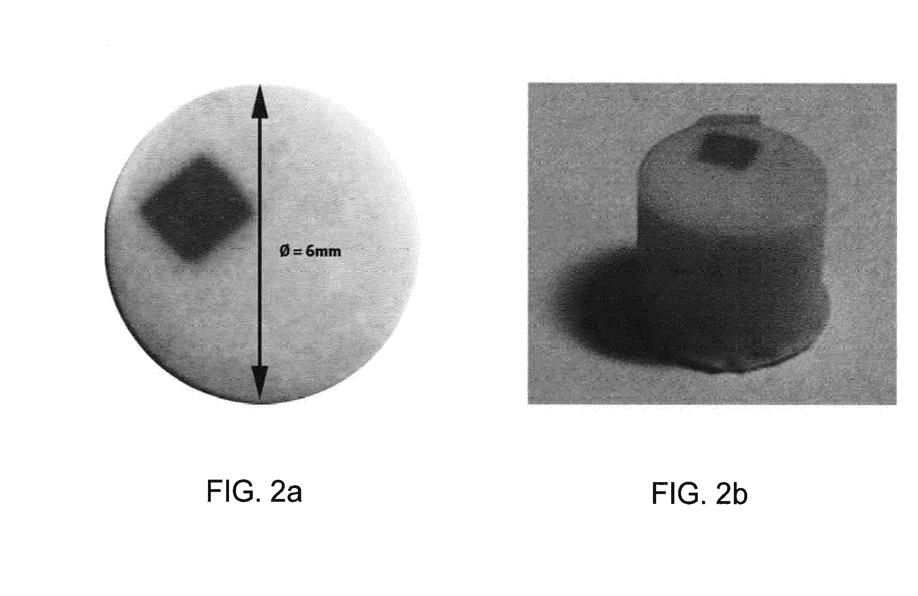

Figure 2a is a top view of the sample section used for the RBS analysis. The dark square (approx. 1.5x1.5 mm²) indicates the position from which the spectrum was acquired. (b)

Figure 2b is a side view of the sample used for the RBS analysis. The area of spectrum acquisition is located on the surface exposed during cutting of the sample.

For the RBS experiments a cross section of a ZrO₂ rod sample was examined. The ZrO₂ rod was cut at a 90° angle with respect to the length axis at a position of approximately 5 mm from the one end of the rod. Before the sample was cut it had been treated by the method of the invention, e.g. soaking in SrCl₂ and subjected to washout experiments for a period of 7 days. The concentration of SrCl₂ used in the preparation was 2M. The prepared sample and the location from which the spectrum was acquired can be found in Figure 2a and figure 2b.

RBS measurements have been carried out in order to determine if the Strontium incorporation when soaking in a SrCl₂ solution is limited to the surface or if the incorporation affects the bulk of the material as well. 2 MeV He²⁺ particles were chosen as probe. This meant a depth of penetration of a few microns.

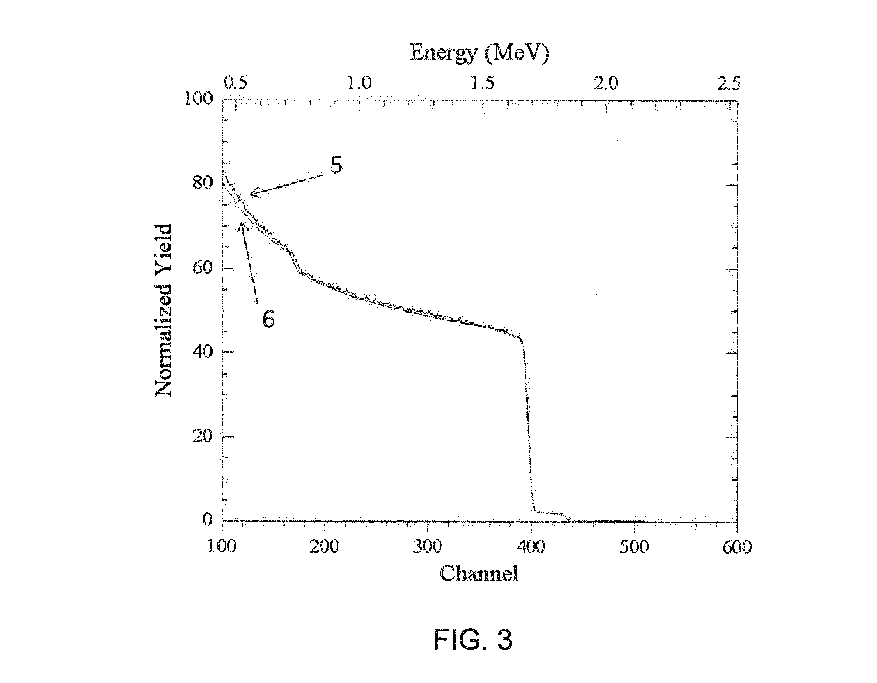

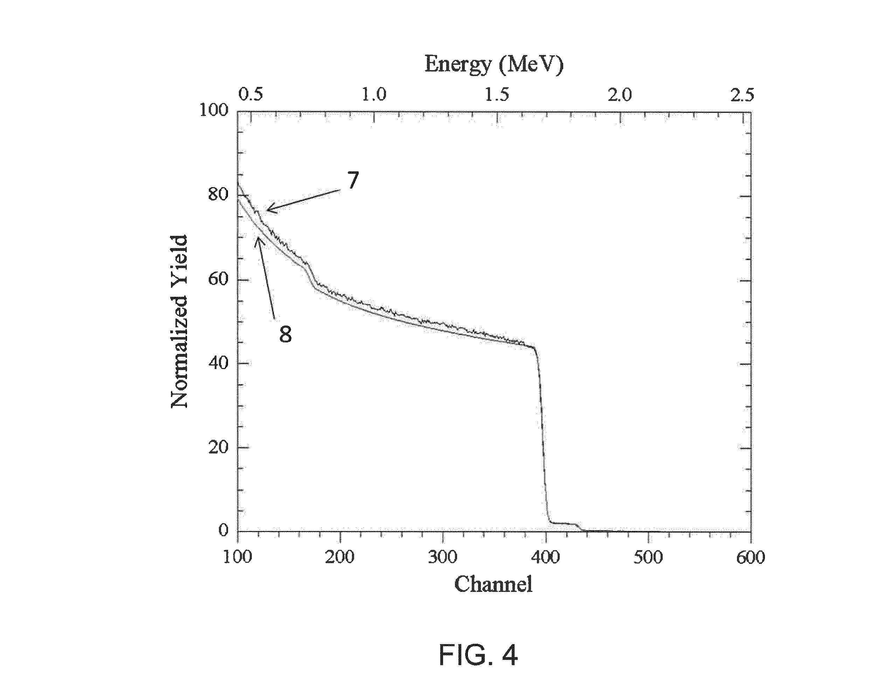

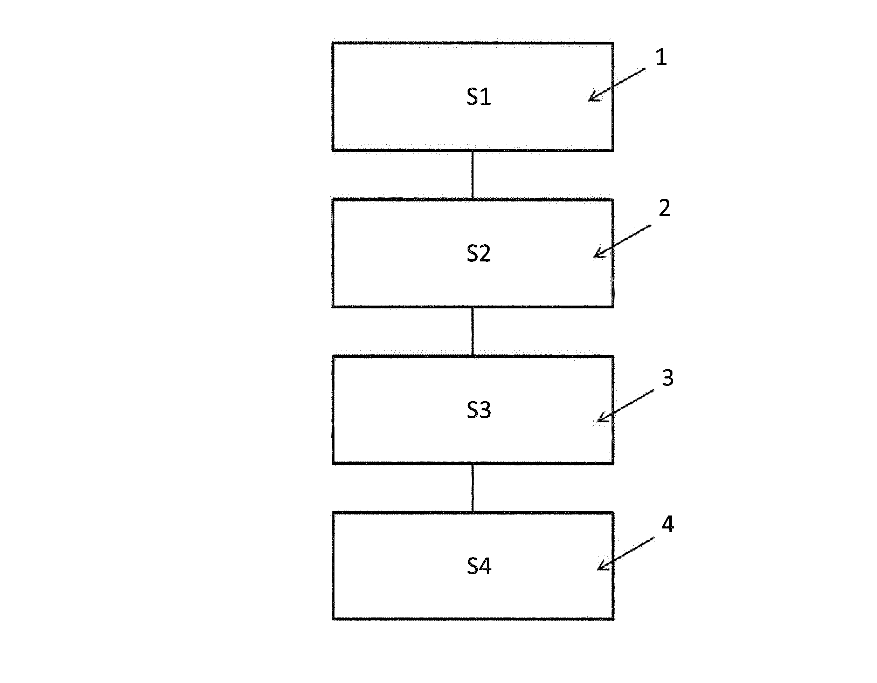

Figure 3 is a graphic plot of data from the RBS measurement and simulation. The top curve marked "5" is the measured data and the bottom curve marked "6" is drawn using data from the simulation. This simulation assumes that the surface concentration of Sr is lower than in the bulk due to washout of Sr during the sample preparation. The simulation was run with a surface Sr concentration of 0.01 at.% and with a bulk of 0.07 at.%. Figure 4 is a graphic plot of data from the RBS measurement and simulation. The top curve marked "7" is the measured data and the bottom curve marked "8" is data from the simulation. This simulation assumes that the surface Sr concentration and the bulk concentration are both 0.01 at.%.

Figure 3 and Figure 4 show the data obtained at the area shown in Figure 2a and figure 2b. The small "foot" from Channel 400 to 440 fits with the presence of tungsten (W). The origin of this is currently under examination. Several simulations were carried out using the simulation program RUMP. This was done in order to determine the material composition that would give rise to the acquired spectrum. Two of these simulations are shown in Figure 3 and 4 as bottom curves 6 and 8. The simulation that fitted the acquired data best assumed that the main constituents of the material were O and Zr at a ratio of 2:1. Then it was assumed that the surface concentration of Sr was lower than that of the bulk due to Sr being washed from the surface by the water used during the cutting procedure. The Sr surface concentration was set at 0.01 at.% in the outer 100 nm and the bulk was set to 0.07 at.%. These data are presented in Figure 3. From the graphs it is evident that the simulation reflects what is actually measured by RBS. From further simulations it is evident that Sr is not only present on the surface exposed by cutting of the sample. An example on such a simulation is shown in Figure 4. Here the bulk concentration of Sr is decreased to match the 0.01 at.% of the surface concentration which causes the two graphs to separate from each other.

Figure 5 is a flow-chart of a method according to the invention.

The method for incorporating alkali metals or alkaline earth metals in a metal oxide according to the invention comprises the steps:
- S1, contacting 1 an aqueous solution comprising alkali metals or alkaline earth metals with a metal oxide body;
- S2 washing 2, e.g. rinsing, the metal oxide body that was in liquid contact with said aqueous solution comprising alkali metals or alkaline earth metals with water or with a second aqueous solution, thereby removing any residual alkali metals or alkaline earth metals based compound that might have been deposited onto said surface of the metal oxide body;
- S3 drying 3 the metal oxide body;
- S4 heating 4 the dried metal oxide body at temperature higher than 1000 °C.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A method for incorporating alkali metals or alkaline earth metals in a metal oxide, the method comprising:
a) contacting an aqueous solution comprising alkali metals or alkaline earth metals with a metal oxide body;
b) drying said metal oxide body;
c) heating said dried metal oxide body at temperature higher than 1000 °C.

2. A method according to claim 1 wherein said alkaline earth metal is strontium.

3. The method according to any of claims 1-2 wherein said contacting is achieved by providing said aqueous solution comprising alkali metals or alkaline earth metals to at least one surface of said metal oxide body so that said aqueous solution comprising alkali metals or alkaline earth metals is in liquid contact with said at least one surface of said metal oxide body.

4. The method according to any of the preceding claims wherein said drying is achieved by drying said at least one surface of said metal oxide body that was in liquid contact with said aqueous solution comprising alkali metals or alkaline earth metals.

5. The method according to any of the preceding claims wherein said drying further comprising placing said metal oxide body that was in liquid contact with said aqueous solution comprising alkali metals or alkaline earth metals under reduced pressure.

6. The method according to any of the preceding claims further comprising: a1) washing said metal oxide body that was in liquid contact with said aqueous solution comprising alkali metals or alkaline earth metals with water or with a second aqueous solution, thereby removing any residual alkali metals or alkaline earth metals based compound that might have been deposited onto said surface of said metal oxide body.

7. The method according to any of the preceding claims wherein said aqueous solution comprising alkali metals or alkaline earth metals, contains alkali metals or alkaline earth metal salts.

8. The method according to claim 7 wherein the concentration of alkali metals or alkaline earth metals in said aqueous solution comprising alkali metals or alkaline earth metals is between 0.001 M and 2M at room temperature.

9. The method according to any of the preceding claims wherein said metal oxide is a ceramic material, such as ZrO₂.

10. The method according to any of the preceding claims wherein said metal oxide body is a body implant.

11. The method according to any of the preceding claims wherein contacting said aqueous solution comprising alkali metals or alkaline earth metals with said metal oxide body comprises keeping said metal oxide body in liquid contact with said aqueous solution comprising alkali metals or alkaline earth metals under reduced pressure.

12. The method according to any of the preceding claims further comprising: d) rinsing said metal oxide body.

13. A ceramic body, comprising
- a metal oxide body comprising alkali metals or alkaline earth metals in said metal oxide body, wherein the concentration of said alkali metals or alkaline earth metals in the bulk is higher than the one at the external surface of said ceramic body.

14. A ceramic body according to claim 13 wherein the concentration in the bulk is between 2 and 10 times higher than the one at the external surface of said ceramic body.

15. A ceramic body, according to any of claims 13-14 comprising
- a metal oxide body comprising alkali metals or alkaline earth metals in said metal oxide body having a structure **characterized by** a concentration gradient of said alkali metals or alkaline earth metals throughout said metal oxide body.
